# EUROPÄISCHE PATENTSCHRIFT

(11) **EP 1 221 319 B1**
(45) Veröffentlichungstag und Bekanntmachung des Hinweises auf die Patenterteilung: **06.05.2004**
(21) Anmeldenummer: 01100241.7
(22) Anmeldetag: 03.01.2001
(51) Int. Cl.: A61M 1/06

(54) **Brustpumpe**
Breast pump
Pompe tire-lait

(43) Veröffentlichungstag der Anmeldung: 10.07.2002
(73) Patentinhaber: Medela AG, 6340 Baar (CH)
(72) Erfinder: Greter, Andy, 6312 Steinhausen (CH); Weber, Beda, 6330 Cham (CH)
(74) Vertreter: Clerc, Natalia

(56) Entgegenhaltungen:
- WO-A-00/66195
- DE-A- 3 916 699
- US-A- 4 961 726

## Beschreibung

Die vorliegende Erfindung betrifft eine Brustpumpe mit einer Membran-Vakuumpumpe mit Antriebsmotor und einer Steuerelektronik, um in Abhängigkeit eines vorprogrammierten Vakuumverlaufs die Motordrehzahl einzustellen, insbesondere eine batteriebetriebene Brustpumpe.

Es sind derzeit Brustpumpen in Entwicklung, welche entsprechend einem wählbaren Programm betrieben werden. Dabei ist in der Regel am Beginn eine Stimulation vorgesehen, bei welcher kurzzeitig bei reduziertem Vakuum (z.B. 6666 - 20000 Pa (50 - 150 mm Hg)), aber hoher Frequenz (z.B. 120 Zyklen/Min.) auf die Brust eingewirkt wird, um die folgende Milchleistung zu stimulieren.

Das eigentliche Milchabpumpprogramm arbeitet bei einem höheren Vakuum (z.B. 13332 - 33330 Pa (100 - 250 mm Hg)) bei wesentlich geringeren Zyklen (z.B. 40 - 80 Zyklen/Min.). Als Vakuumpumpe dient eine Membranpumpe. Das gewünschte Vakuum wird über die Einstellung der Drehzahl des Antriebsmotors der Pumpe geregelt. Das Programm kann am Ende des Absaugens noch eine Auslaufphase beinhalten (Zurückschalten des Vakuums auf z.B. 6666 - 20000 Pa (50 - 150 mm Hg) bei z.B. 120 - 150 Zyklen/Min.).

Ein besonders vorteilhaftes Absaugprogramm besteht bei jedem Zyklus im progressiven Aufbau des gewünschten Vakuums und Halten des maximalen Vakuums während einer vorbestimmten Zeit und anschliessend einem möglichst raschen vollständigen Abbau des Vakuums (auf Null-Vakuum), um dann nach einer vorbestimmten Ruhepause den folgenden Zyklus in Angriff zu nehmen.

WO 00/66195 offenbart eine Brustpumpe mit einem Antriebsmotor, einem Vakuumsensor und einem Magnetventil, welches eine zur Atmosphäre hin offene Bypassleitung öffnet. Durch Regelung des Motors lassen sich die Pumpzyklen variieren.

DE 39 16 699 beschreibt eine Brustpumpe mit einem Milchgefäss, einem damit über eine Leitung verbundenen kleineren Zwischengefäss und einer mit dem Zwischengefäss über eine Leitung verbundenen Ventilanordnung. Die Ventilanordnung ermöglicht ein schnelles Absinken des Unterdrucks.

Aufgabe der vorliegenden Erfindung ist es, bei einer Brustpumpe der eingangs definierten Art Mittel vorzusehen, welche eine möglichst rasche Vakuumfreigabe (vacuum release) zwischen dem Ende des "Vakuumhaltens" und der "Ruhepause bei Null-Vakuum" erlauben.

Diese Aufgabe wird bei der eingangs definierten Brustpumpe erfindungsgemäss mit den Merkmalen gemäss dem kennzeichnenden Teil von Anspruch 1 gelöst.

Besondere Ausführungsformen des Erfindungsgegenstandes sind in den abhängigen Ansprüchen definiert.

Der Erfindungsgegenstand wird nachstehend anhand von in den Zeichnungen gezeigten Ausführungsbeispielen noch etwas näher erläutert. Es zeigt:
- Fig. 1: rein schematisch den programmierten Vakuumverlauf bei einer Brustpumpe;
- Fig. 2: das Blockschema einer erfindungsgemäss aufgebauten Brustpumpe, und
- Fig. 3: eine ähnliche Darstellung wie Fig. 2, mit einer Variante der gesteuerten Einrichtung zur Aufhebung des erzeugten Vakuums.

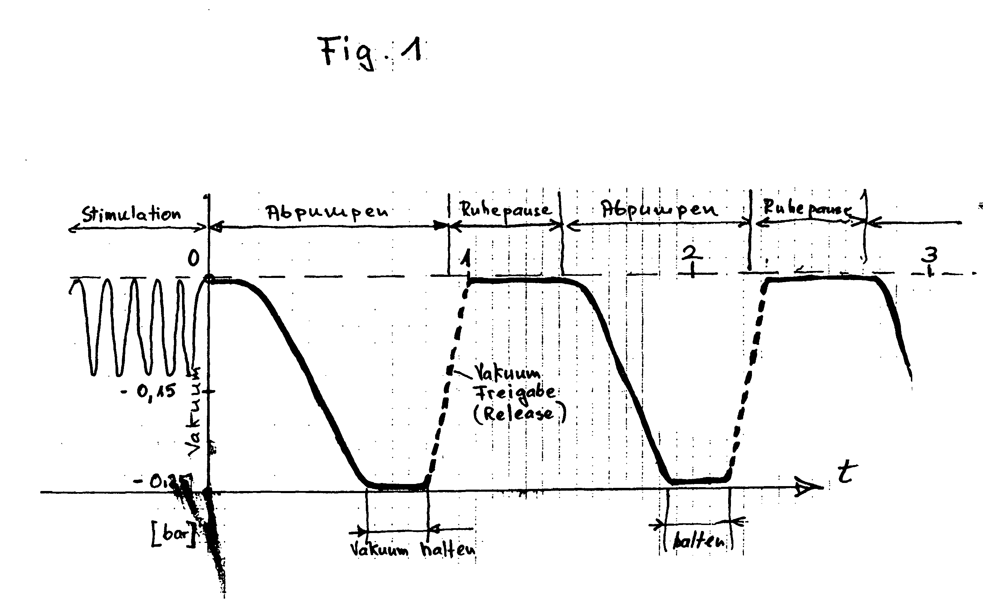

Figur 1 der Zeichnung zeigt schematisch den programmierten Verlauf des Vakuums beim Betrieb einer Brustpumpe, wobei nach einer eventuellen Stimulation bei einem Vakuum von 6666 - 20000 Pa (50 - 150 mm Hg) bei z.B. 120 Zyklen/Min. mit dem eigentlichen Abpumpen begonnen wird. Angestrebt wird ein Vakuum von 33 330 Pa (250 mm Hg), welches von 0 bis auf den Endwert progressiv aufgebaut wird, und welches während einer gewissen Zeit pro Zyklus aufrechterhalten wird und danach möglichst rasch wieder auf den Wert 0 abgebaut wird. Dort wird eine kurze Ruhepause eingeschaltet, bevor der nächste Zyklus gestartet wird (das eigentliche Abpumpen erfolgt z.B. bei 40 - 80 Zyklen/Min.).

Der Aufbau des gewünschten Vakuums erfolgt über eine elektronische Drehzahlregelung des Elektromotors für die Vakuumpumpe (Membranpumpe). Die Pumpe wird für das Erreichen eines bestimmten Vakuums mit einer vorbestimmten Drehzahl angetrieben, welche während dem Abpumpvorgang beibehalten wird. Der zwischen den Zyklen eingeschaltete Vakuumabbau und die anschliessende Ruhepause erfolgt nicht durch Veränderung der Motordrehzahl, sondern durch Vakuumfreigabe (Abbau) in einer zwischen Pumpe und Absaughaube geschalteten Vakuumkammer mit Trennmembran. Die Vakuumfreigabe erfolgt über eine gesteuerte Einrichtung, welche das Vakuum in der Vakuumkammer durch passive Ventilation (Ventil zur Umgebung) oder aktive Druckventilation (Druckaufbau in der Vakuumkammer durch Zuschaltung der Ausstossleitung der Vakuumpumpe) raschmöglichst abbaut (ohne die Pumpe anzuhalten).

Durch geeignete Einstellung der Motordrehzahl des Pumpenantriebes lässt sich ein gewünschter Verlauf des Vakuums beim Abpumpen einstellen (programmieren), d.h. die Steuerung der Zyklen und des angestrebten maximalen Vakuums. Die Ruhepause lässt sich durch Steuerung der Einrichtung zur Aufhebung des aufgebauten Vakuums ebenfalls einstellen.

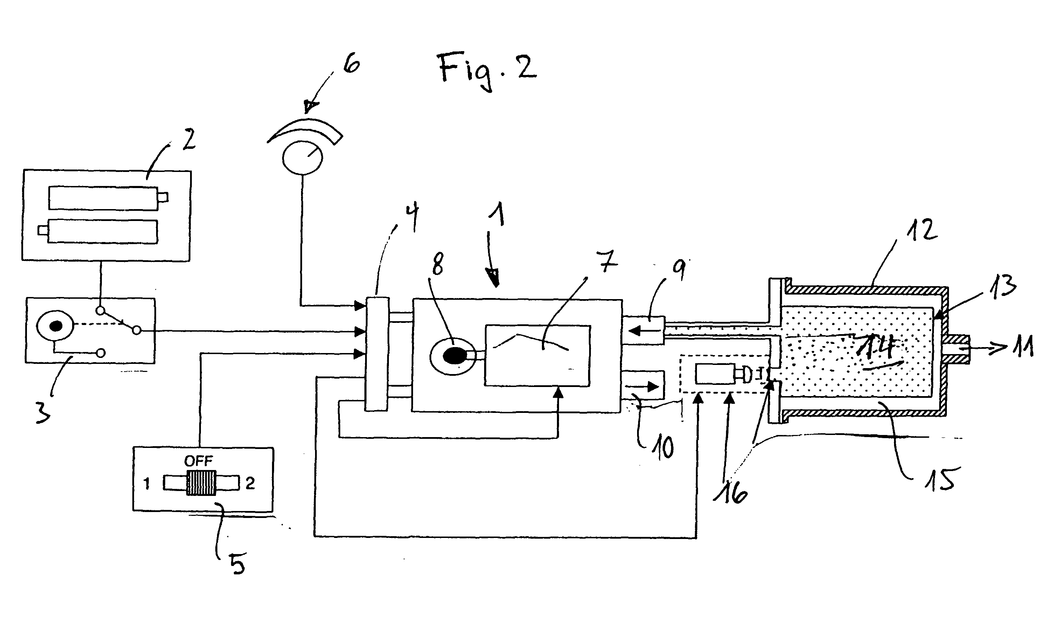

Figur 2 der Zeichnung zeigt rein schematisch den Aufbau einer erfindungsgemässen batteriebetrieben Brustpumpe.

Das Aggregat, d.h. die eigentliche Vakuumpumpe, ist eine an sich bekannte Membran-Vakuumpumpe 1, welche von Batterien 2 über einen Hauptschalter 3 in Betrieb genommen wird.

Eine Steuerelektronik 4 nimmt einerseits Befehle eines Programms für den gewünschten Kurvenverlauf beim Abpumpen entgegen (Gerät 5), sowie solche einer manuellen Einstellung (Gerät 6) (Verstellung) des Vakuums und der Zyklen entgegen und wirkt direkt auf die zum Erreichen der Werte erforderliche Motordrehzahl ein.

Der Motor 7 treibt über einen Kurbeltrieb 8 eine Membranpumpe an, welche über die Leitung 9 ein Vakuum erzeugt und abgesaugte Luft über die Leitung 10 ausstösst.

Als Membran-Vakuumpumpe eignet sich wegen ihrer Kompaktheit und Effizienz insbesondere eine solche, welche Gegenstand einer gleichzeitig eingereichten Patentanmeldung der Anmelderin bildet.

Zwischen einer Absaughaube 11 (nicht dargestellt) und der Vakuumpumpe 1 (Aggregat) ist eine Vakuumkammer 12 mit Trennmembran 13 geschaltet. Die erste Kammer 14 der zugeschalteten Vakuumkammer 12 steht mit der Saugleitung 9 der Pumpe 1 in Verbindung, während die mittels Membran 13 (Diaphragma) abgetrennte Kammer 15 mit der Absaughaube 11 verbindbar ist.

Die erste Kammer 14 ist mit einem gesteuerten Ventil 16 ausgerüstet, welches nach Öffnung das aufgebaute Vakuum unverzüglich abbaut (Vakuumfreigabe in der Abpumpkurve nach Figur 1). Der Vakuumabbau erfolgt somit durch passive Ventilation der ersten Kammer der Vakuumkammer (damit baut sich gleichzeitig auch das Vakuum in der zweiten Kammer und somit in der Absaughaube ab).

Die Steuerung des Ventils 16 erfolgt nach Massgabe des gewählten Programms über die Steuerelektronik 4.

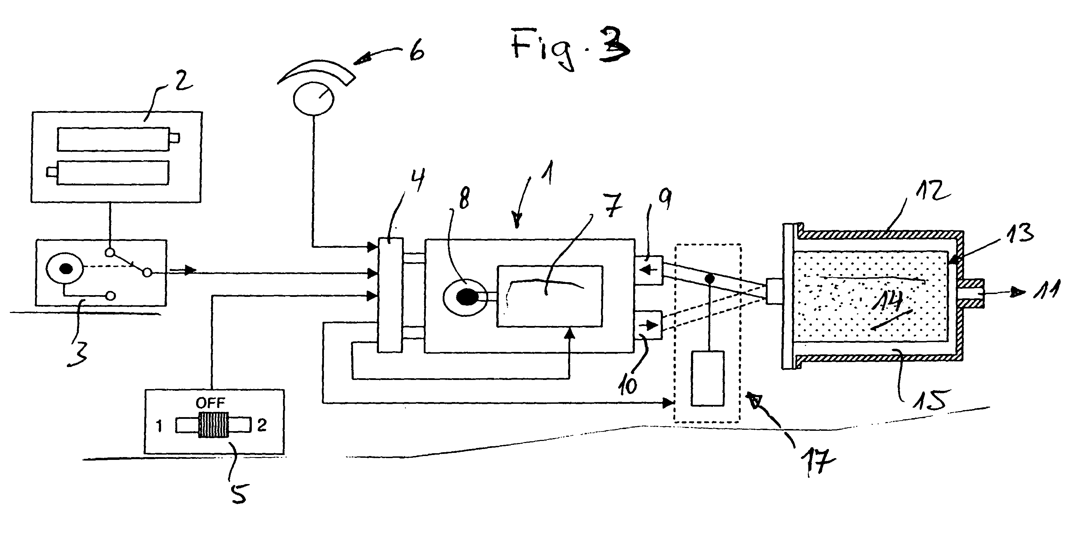

Eine Variante der Brustpumpe nach Figur 2 ist ebenfalls schematisch in Figur 3 dargestellt. Der Unterschied zur Ausführungsform nach Figur 2 liegt in der Einrichtung zur Aufhebung des in der Vakuumkammer 12 aufgebauten Vakuums: Anstelle des für eine passive Ventilation vorgesehenen gesteuerten Ventils 16 ist hier eine sogenannte Ventilweiche 17 vorgesehen, welche die Verbindung zwischen der Saugleitung 9 und der ersten Kammer 14 unterbricht und dafür die Ausstossleitung 10 der Pumpe mit der Kammer 14 verbindet. Damit wird das in der Kammer 14 (und damit in der Kammer 15 und der Absaughaube 11) erzeugte Vakuum durch positiven Druckaufbau aufgehoben. Dies ist deshalb innert kürzester Zeit möglich, weil die Pumpe auch während der Vakuumfreigabe (Release) und der folgenden Ruhepause weiterarbeitet. Der Befehl zum Umschalten der Ventilweiche erfolgt ebenfalls über die programmierte Steuerelektronik 4.

Kernstück der Brustpumpe ist neben der vorteilhafterweise miniaturisierten Membranpumpe die zugeschaltete Vakuumkammer 12, auch Medium-Trennkammer genannt. Dank dieser Kammer kann das aufgebaute Vakuum ohne Unterbruch der eigentlichen Vakuumpumpe jederzeit (programmgesteuert) aufgehoben werden und unverzüglich wieder aufgebaut werden.

## Patentansprüche

1. Brustpumpe mit einer Membran-Vakuumpume (1) mit Antriebsmotor (7) und einer Steuerelektronik (4), um in Abhängigkeit eines vorprogrammierten Vakuumverlaufs die Motordrehzahl einzustellen, **dadurch gekennzeichnet, dass** zusätzlich eine Vakuumkammer (12) mit Trennmembran (13) vorgesehen ist, deren erste durch die Trennmembrane (13) abgetrennte Kammer (14) mit der Saugleitung (9) der Vakuumpumpe (1) in Verbindung steht und deren zweite durch die Trennmembrane (13) abgetrennte Kammer (15) mit einer Absaughaube (11) verbindbar ist, wobei die erste Kammer (14) mit einer gesteuerten Einrichtung (16, 17) zur Aufhebung des aufgebauten Vakuums ausgerüstet ist.

2. Brustpumpe nach Anspruch 1, **dadurch gekennzeichnet, dass** die Einrichtung zur Aufhebung des Vakuums in der Vakuumkammer (12) aus einem von der Steuerelektronik (4) gesteuerten Ventil (16) besteht, welches bei Öffnung die erste Kammer (14) mit der Umgebung verbindet, d.h. die Vakuumkammer (12) passiv entlüftet.

3. Brustpumpe nach Anspruch 1, **dadurch gekennzeichnet, dass** die Einrichtung zur Aufhebung des Vakuums in der Vakuumkammer (12) aus einer Ventilweiche (17) besteht, welche bei Betätigung die Saugleitung (9) zwischen Vakuumpumpe (1) und der ersten Kammer (14) unterbricht und dafür die genannte erste Kammer (14) der Vakuumkammer (12) mit einer einen positiven Druck aufbauenden Ausstossleitung (10) der Vakuumpumpe (1) verbindet, d.h. das Vakuum in der Vakuumkammer (12) durch positiven Druckaufbau aktiv aufhebt.

## Claims

1. A breast pump with a diaphragm vacuum pump (1) with a drive motor (7) and a control electronics (4) for setting the motor speed as a function of a preprogrammed vacuum profile, **characterized in that** a vacuum chamber (12) with a separating diaphragm (13) is additionally provided, the first chamber (14) of which vacuum chamber communicates with the suction line (19) of the vacuum pump (1), and the second chamber (15) of which can be connected to the suction cap, this first chamber being separated by the separating diaphragm (13) and this second chamber being separated by the separating diaphragm (13), the first chamber (14) being equipped with a controlled device (16, 17) for canceling the vacuum which has been built up.

2. The breast pump as claimed in claim 1, **characterized in that** the device for canceling the vacuum in the vacuum chamber (12) consists of a valve (16) which is controlled by the control electronics (4) and which, when opened, connects the first chamber (14) to the environment, i.e. passively removes air from the vacuum chamber (12).

3. The breast pump as claimed in claim 1, **characterized in that** the device for canceling the vacuum in the vacuum chamber (12) consists of a valve switch (17) which, when actuated, interrupts the suction line (9) between the vacuum pump (1) and the first chamber (14) and connects said first chamber (14) of the vacuum chamber (12) to the ejection line (10) of the vacuum pump (1) building up a positive pressure, i.e. actively cancels the vacuum in the vacuum chamber (12) by positive pressure buildup.

## Revendications

1. Tire-lait comprenant une pompe à vide à membrane (1) avec un moteur d'entraînement (7) et une électronique de commande (4), afin d'ajuster la vitesse du moteur en fonction d'une progression du vide préprogrammée, **caractérisé en ce qu'**il est prévu en outre une chambre à vide (12) avec une membrane de séparation (13) dont la première chambre (14) séparée par la membrane de séparation (13) est en liaison avec la conduite de succion (9) de la pompe à vide (1) et dont la deuxième chambre (15) séparée par la membrane de séparation (13) peut être connectée à une cloche de succion (11), la première chambre (14) étant équipée d'un dispositif commandé (16, 17) pour supprimer le vide créé.

2. Tire-lait selon la revendication 1, **caractérisé en ce que** le dispositif pour supprimer le vide dans la chambre à vide (12) se compose d'une valve (16) commandée par l'électronique de commande (4) qui, lors de son ouverture, connecte la première chambre (14) à l'environnement, c'est-à-dire purge passivement la chambre à vide (12).

3. Tire-lait selon la revendication 1, **caractérisé en ce que** le dispositif pour supprimer le vide dans la chambre à vide (12) se compose d'un aiguillage de valve (17) qui, lors de son actionnement, interrompt la conduite de succion (9) entre la pompe à vide (1) et la première chambre (14) et pour cela relie ladite première chambre (14) de la chambre à vide (12) à une conduite d'échappement (10) de la pompe à vide (1) créant une pression positive, c'est-à-dire supprimant activement le vide dans la chambre à vide (12) par une augmentation de pression positive.
